# EUROPEAN PATENT SPECIFICATION

(11) **EP 1 098 882 B1**
(45) Date of publication and mention of the grant of the patent: **25.08.2004**
(21) Application number: 99926713.1
(22) Date of filing: 18.06.1999
(51) Int. Cl.: C07D 221/16

(54) **A PROCESS FOR PREPARING BENZOCYCLOHEPTAPYRIDIN-11-ONES**
VERFAHREN ZUR HERSTELLUNG VON BENZOCYCLOHEPTAPYRIDIN-11-ONE
PROCEDE PERMETTANT DE PREPARER DES BENZOCYCLOHEPTAPYRIDIN-11-ONES

(30) Priority: 24.07.1998 IE 980619; 18.09.1998 IE 980776
(43) Date of publication of application: 16.05.2001
(73) Proprietor: RUSSINSKY LIMITED, Cork (IE)
(72) Inventor: SCHICKANEDER, Helmut, Cork (IE); NIKOLOPOULOS, Aggelos, Cork (IE); KOCHER, Christian, Cork (IE); MULCAHY, David, Cork (IE)
(74) Representative: O'Brien, John Augustine
(86) International application number: PCT/IE1999/000054
(87) International publication number: WO 2000/005215

(56) References cited:
- EP-A- 0 208 855
- EP-A- 0 288 640
- EP-A- 0 396 083
- WO-A-98/11092
- CH-A- 688 412
- US-A- 3 357 986

## Description

### Introduction

(Benzocycloheptapyridinylidene) piperidine derivatives of the general formula (A) are known as non-sedating histamine H₁-receptor antagonists, PAF (platelet activating factor) antagonists or FPT (farnesyl protein transferase) inhibitors:

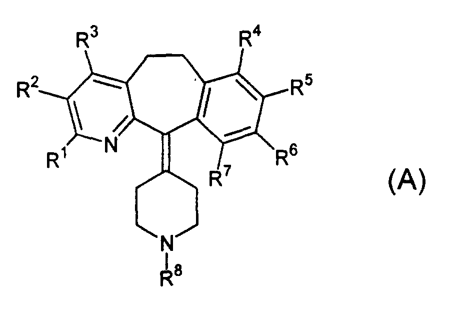

The substituents R¹, R², R³, R⁴, R⁵, R⁶ and R⁷ may be chosen individually from the following groups:
hydrogen, halogen, hydroxy, C₁-C₆-alkyl, C₁-C₆-alkoxy, nitro, CF₃, thio-C₁-C₆-alkyl.

The substituent R⁸ may be represented by:
hydrogen, C₁-C₆-alkyl or COOR⁹ (wherein R⁹ may be C₁-C₆-alkyl, C₁-C₆-alkenyl, phenyl, benzyl), CO-aryl, CO-heteroaryl, CO-alkyl, CO-alkyl-aryl.

For the synthesis of compounds of type (A) benzocycloheptapyridin-11-ones of formula (B) are frequently used as key intermediates:

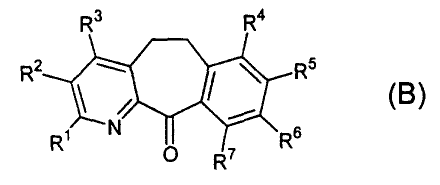

The substituents R¹, R², R³, R⁴, R⁵, R⁶ and R⁷ in formula (B) are as defined above for compounds of formula A.

Various routes for obtaining compounds of formula (B) are discussed in J. Med. Chem. *34*,457-461 [1991]; Bioorg. Med. Chem. Lett. *3*,1073-1078 [1993]; EP-A-0 208 855 and CH -A- 688 412.
It is known to use a 2-cyano-3-methyl pyridine derivative (I) as a starting material to prepare the tricyclic key intermediate (B) (Scheme 1). The nitrile (I) is first protected as the *tert*.-butyl amide (II) via a Ritter-type reaction. The amide thus obtained is then reacted with at least two equivalents of *n*-butyl lithium affording a dianion. Subsequent alkylation of the generated dianion with a benzylhalide provides a compound of formula (III).

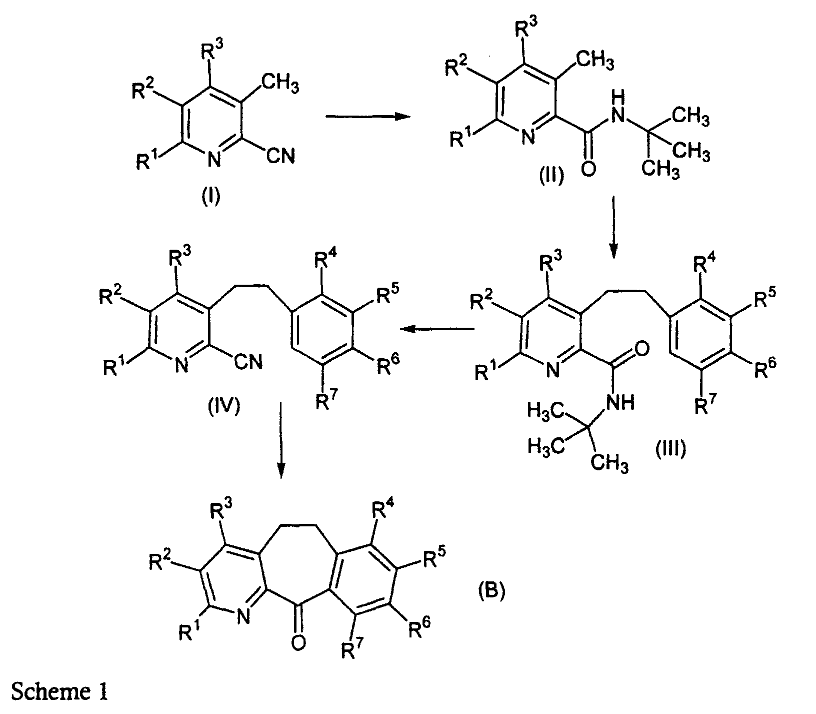

After deprotection of coupling product (III) the obtained nitrile (IV) is cyclised under acidic conditions to give key intermediate (B).

The disadvantage of this reaction sequence is that the deprotonation and coupling reaction for producing a compound of type (III) has to be carried out at very low temperature in the range -50°C to -30°C with at least two equivalents of *n*-butyl lithium.

Such low temperature reactions with *n*-butyl lithium are hazardous and expensive to perform, especially on a factory scale. In addition protection-deprotection procedures cause additional costs and lower the efficiency of the process. Moreover environmental aspects have to be considered with each performed reaction step.

Therefore there is the need for a simple and efficient process which can be performed in a convenient temperature range. Furthermore there is the necessity of an atom-efficient and environmentally friendly process requiring only a minimum of hazardous chemicals.

### Statements of the invention

According to the invention there is provided a simple and atom-efficient process for the preparation of benzocycloheptapyridin - 11 - ones of the general formula B: 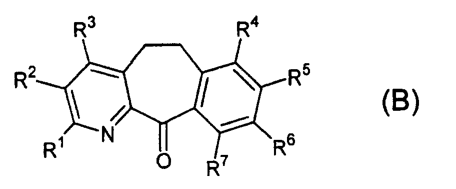 wherein R¹, R², R³, R⁴, R⁵, R⁶ and R⁷ are independently selected from one or more of the following groups:
hydrogen, halogen, hydroxy, C₁-C₆-alkyl, C₁-C₆-alkoxy, nitro, CF₃, thio-C₁-C₆-alkyl,
and pharmaceutically acceptable salts thereof, comprising the steps of:-
deprotonation of a compound of the formula V or a compound of the formula VI: 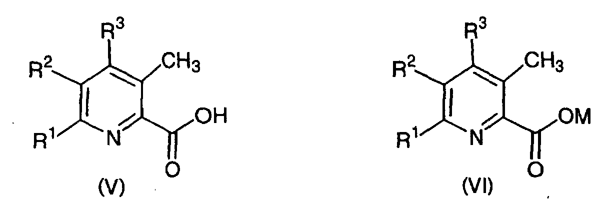 wherein R¹, R² and R³ are as defined above, and M is an alkali metal ion.
to form a dianion;
alkylation of the dianion to form a coupling intermediate VII. 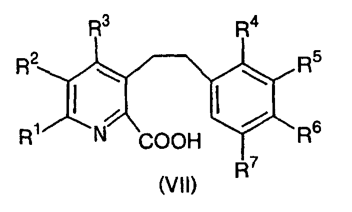 wherein R¹, R², R³, R⁴, R⁵, R⁶ and R⁷ are as defined above; and
converting the coupling intermediate VII into a compound of formula B via the acid chloride and carrying out a Friedel Crafts reaction.

In one embodiment of the invention deprotonation is carried out using a lithium deprotonation agent. Preferably the lithium deprotonation agent is a lithium amide or n-butyl lithium.

Particularly preferred is a process wherein deprotonation is carried out by reaction of a compound of formula VI with one equivalent of the deprotonation agent.

In another instance deprotonation is carried out by reaction of a compound of formula V with two equivalents of the deprotonation agent. Preferably the lithium amide is LDA (lithium diisopropylamide).

In a preferred embodiment of the invention the dianions are picolinic acid dianions generated from picolinic acid or salts thereof, especially the lithium salt.

Preferably the reaction is carried out within a temperature range of from -50°C to 70°C, most preferably within a temperature range of from -15°C to 20°C.

Ideally the process is carried out in the presence of a solvent. The preferred solvent is a donor solvent, especially THF (tetrahydrofuran).

Preferably alkylation involves the addition of benzylhalide to the dianion. Ideally the alkylation reaction is carried out within a temperature range of from -50°C to 70°C, most preferably within a temperature range of from -15°C to 20°C.

Ideally the alkylation process is carried out in the presence of a solvent, preferably a donor solvent, especially tetrahydrofuran.

In one embodiment of the invention compound B is a benzocycloheptapyridin-11-one.

In another embodiment of the invention the process includes the step of preparing (benzocycloheptapyridinylidene) piperidine derivatives of formula A: 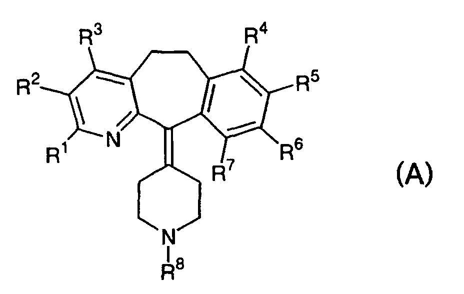 wherein R¹, R², R³, R⁴, R⁵, R⁶ and R⁷ are as defined above and R⁸ may be represented by:
hydrogen, C₁-C₆-alkyl or COOR⁹ (wherein R⁹ may be C₁-C₆ -alkyl, C₁-C₆ - alkenyl, phenyl, benzyl), CO-aryl, CO-heteroaryl, CO-alkyl, CO-alkyl-aryl.

Another embodiment of the invention is the use of benzocycloheptapyridin-11-ones for the preparation of antihistaminics, PAF antagonists and FPT inhibitors.

### Description of the Invention

The invention will be more clearly understood from the following description thereof, given by way of example only.

A novel method for generating dianions using 3-methylpyridine-2-carboxylic acid derivatives of formula (V) or using their corresponding alkali metal salts of formula (VI), preferably from their lithium salt (Scheme 3) is described.

The substituents R¹, R², R³, R⁴, R⁵, R⁶ and R⁷ in Scheme 3 are defined as herein described above. Preferably the substituents are selected from one or more of hydrogen, halogen or C₁-C₆ alkyl groups. M is represented by any alkali metal ion, preferably lithium.

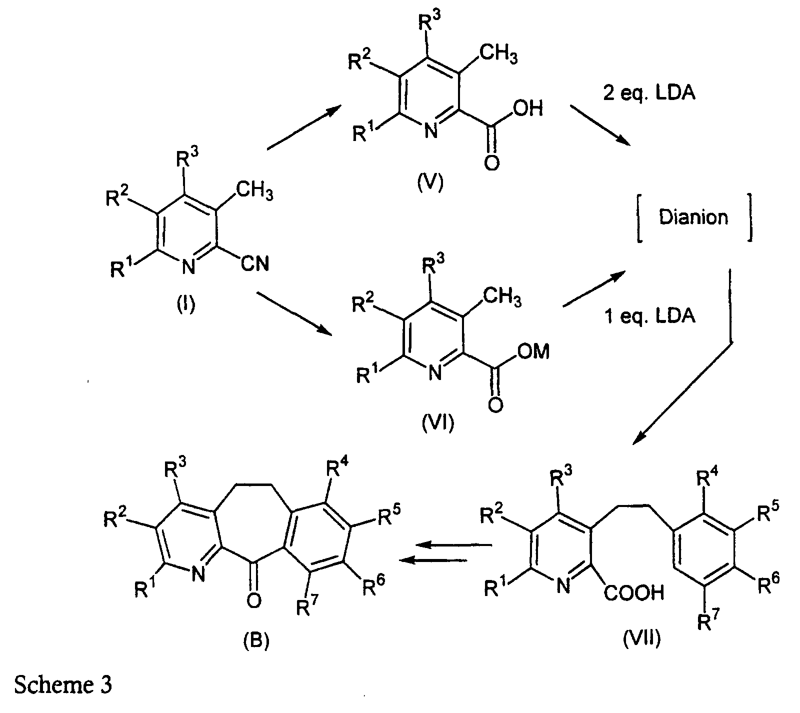

A 2-cyano-3-methyl pyridine of formula (I) is hydrolysed to give a carboxylic acid derivative of type (V) or the corresponding alkali salt (VI). The alkali salt (VI), preferably the lithium salt is reacted with only one equivalent of *n*-butyl lithium or lithium amide, preferably LDA (lithium diisopropylamide) to form the dianion. When reacting the free acid (V) in an analogous way two equivalents of deprotonation reagent are necessary to generate the dianion. In both cases it was found that the dianion is surprisingly stable in a temperature range from below 0°C up to room temperature and above.
Under these mild conditions a C-alkylation reaction can be successfully performed in very good yield (>70%) by adding a benzylhalide to the dianion solution. The product (VII) is isolated in high quality (purity >80%) and may be further purified by re-crystallisation.
Final conversion of the coupling intermediate (VII) into ketone (B) is achieved by reacting the acid (VII) into its hydrochloride and further into the acid chloride for performing a Friedel-Crafts reaction. A good overall yield for these final steps is obtained (>65%).

In general it appears to be of particular advantage to generate the alkali carboxylate (VI), most preferably the lithium carboxylate prior to the dianion formation. In this case only one equivalent of LDA is required and the subsequent alkylation can be performed with a minimum of hazardous substances in a very convenient temperature range.

### Example 1

### Preparation of 3-methylpyridine-2-carboxylic acid

251 g (2.13 mol) of 2-cyano-3-methylpyridine are suspended in 700-800 ml of water and 159 g KOH (2.4 mol) are added. The mixture is heated to reflux for 5-6 hours, then the water is distilled off under vacuum. 300-400 ml of acetone are added to the residue. Stirring is continued for 1-2 hours. The precipitate is filtered off and re-crystallised from ethanol. 280 g (76%) of potassium 3-methyl-pyridine-2-carboxylate are obtained. The potassium salt is converted into the free acid by dissolving it in 200-250 ml of water. The suspension is then heated to 30-50°C and 107 g (1.76 mol) of glacial acetic acid are added. The reaction mixture is subsequently cooled to 0-5°C and stirred at this temperature for 1-1.5 hours. The product is filtered off and washed with ethanol affording 170 g (70 %) of 3-methylpyridine-2-carboxylic acid.
m.p.:116.5-118°C.

| Microanalysis C₇H₇NO₂ [137.14] | | | |
|---|---|---|---|
| calc.: | C: 61.3 | H:5.1 | N: 10.2 |
| found.: | C:61.7 | H: 5.26 | N: 10.6 |

¹H-NMR (270.05 MHz, D₆-DMSO): δ = 2.49 (s, 3H, CH₃), 7.49 (dd, 1H, H-5), 7.79 (dd, 1H, H-4), 8.49 (d, 1H, H-6), 10.72 (s_{br}, 1H, CO₂H).
¹³C-NMR (67.8 MHz, D₆-DMSO): δ = 19.29 (CH₃), 126.11 (C-5), 133.77 (C-3), 139.89 (C-4), 146.62 (C-6), 148.91 (C-2), 167.87 (CO₂H).
FT-IR (KBr): 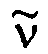 [cm⁻¹] = 3354, 2923, 2201.5, 2142, 1667, 1602, 1520, 1463, 1442, 1360, 1297, 1229, 1164, 1124, 1069, 1001, 958, 854, 810.5, 697.5, 587.

### Example 2

### Preparation of lithium 3-methylpyridine-2-carboxylate

50.0 g (0.42 mol) of 2-cyano-3-methylpyridine are suspended in approximately 200 ml of water and 17.3 g lithium hydroxide monohydrate (0.40 mol) are added. The mixture is heated to reflux for 5-6 hours. Then the water is distilled off under vacuum. 300 ml of acetone are added to the obtained residue and stirring is continued for 1-2 hours. The product is filtered off and dried under vacuum. Yield: 57.8 g (0.40 mol, 96%).
¹H-NMR (60 MHz, D₂O): δ = 2.4 (s, 3H, CH₃), 7.25 (dd, 1H, H-5), 7.65 (d, 1H, H-4), 8.25 (d, 1H, H-6).
FT-IR (KBr): 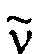 [cm⁻¹] = 3431, 3061.5, 2966, 2926.5, 1648, 1631, 1613, 1571, 1447,1430,1384,1241,1115,868,792,712,670,580,508.

### Example 3

### Preparation of 3-[2-(3-Chlorophenyl)ethyl]-pyridine-2-carboxylic acid using one equivalent of LDA

15.0 g (105 mmol) of lithium 3-methylpyridine-2-carboxylate are suspended in approximately 300 ml of THF under nitrogen atmosphere. The mixture is kept at a temperature of from -30°C to 70°C, preferably -15°C to 30°C and 56.0 ml (112 mmol) of a 2 molar solution of lithium diisopropylamide are added forming a purple solution. Then 15.0 ml (118 mmol) of *m*-chlorobenzyl chloride are added. Stirring is continued at -30°C to 70°C, preferably -15°C to 30°C. The solvent is removed and the obtained solid is washed with petroleum ether, then suspended in water and acidified with glacial acetic acid. The title compound is extracted with MIBK in a yield of 19.2 g (73.4 mmol, 70%).
¹H-NMR (60 MHz, CDCl₃): δ = 2.95 (m, 2H, CH₂), 3.45 (m, 2H, CH₂), 6.9 - 7.7 (m, 6H, H-aryl), 8.5 (s_{br}, 1H, H-6-pyridyl), 11.9 (s, 1H, CO₂H).
FT-IR (KBr): ν [cm⁻¹] = 3431, 3087, 2924, 1642, 1596, 1512, 1501, 1479, 1461, 1450, 1430, 1350, 1326, 1284, 1207, 1086, 892, 872, 843, 796, 780, 699, 654, 620.

### Example 4

### Preparation of 3-[2-(3-Chlorophenyl)ethyl]-pyridine-2-carboxylic acid using two equivalents of LDA

5.0 g (36.5 mmol) of 3-methylpyridine-2-carboxylic acid are dissolved in approximately 200 ml of THF under nitrogen atmosphere. The mixture is kept at -30°C to 70°C, preferably -15°C to 30°C. 38.0 ml (76 mmol) of a 2 molar solution of lithium diisopropylamide are added to form a purple solution. 5.1 ml (6.5 g, 40.2 mmol) of *m*-chlorobenzyl chloride are added. Stirring is continued preferably at a temperature of -15°C to 30°C. The solvent is removed under vacuum and the obtained solid is washed with petroleum ether. The crude product is suspended in water and acidified with glacial acetic acid. The title compound is extracted with MIBK in a yield of 8.6 g (32.9 mmol, 90%).

Optionally the product can be converted into the hydrochloride by treatment with hydrochloric acid.

¹H-NMR (60 MHz, D₆-DMSO): δ = 2.90 (m, 2H, CH₂), 3.45 (m, 2H, CH₂), 7.3 (m, 4H, H-aryl), 7.95 (m, 2H, H-aryl), 8.5 (d, 1H, H-aryl), 8.8 (d, 1H, H-aryl), 12.1 (s, 1H, CO₂H and N-H).
FT-IR (KBr): ν [cm⁻¹] = 3103, 2920, 2868, 2359, 2342, 1800, 1733, 1705, 1622, 1596, 1574, 1538, 1465, 1411, 1275, 1236, 1207, 1184, 1162, 1084, 996, 908, 878, 813,797,749,696,686,647.
Mp.: 175-184°C.

### Example 5

### Preparation of 8-Chloro-6,11-dihydro-5H-benzo-[5,6]-cyclohepta[1,2-b]pyridine-11-one

75.0 g, (0.25 mol) of 3-[2-(3-Chlorophenyl)ethyl]-pyridine-2-carboxylic acid hydrochloride are suspended in approximately 300 ml of thionylchloride. The mixture is stirred in a temperature range of 30-60°C, preferably 40-50°C. After evaporation of the excess of thionylchloride 79.5 g of the carboxylic acid chloride are obtained.

FT-IR (KBr): ν [cm⁻¹] = 3420, 2924, 2854,1749, 1611, 1596, 1571, 1528, 1477, 1458, 1426, I208, 1150, 1077, 896, 880,846, 803, 702, 684, 650, 622.

The material obtained from the previous step is suspended in a suitable solvent such as 1,2-dichloroethane, 70.0 g (0.525 mol) of AlCl₃ are added and the mixture is stirred at -5°C to 20°C, preferably at 0-5°C. After acidification with diluted HCl the aqueous phase is separated and re-basified with 30% NaOH. The product is extracted with a suitable solvent such as toluene and re-crystallised. Yield 39.5 g (0.162 mol, 65%).

| Microanalysis C₁₄N₁₀NOCl (243.69) | | | |
|---|---|---|---|
| Calc | C: 69.00 | H: 4.14 | N: 5.74 |
| Found | C: 68.72 | H: 4.20 | N: 5.34 |

¹H-NMR (400 MHz, CDCl₃): δ = 3.20 (m, 4H, CH₂), 7.32 (m, 3H, H-aryl), 7.63 (m, 1H, H-aryl), 8.04 (d, 1H, H-aryl), 8.68 (m, 1H, H-aryl).
¹³C-NMR (67.8 MHz, CDCl₃): δ = 32.45, 34.50 (CH₂); 125.79, 127.22, 129.53, 132.80, 135.91, 136.48, 137.16, 138.87, 142.01, 148.68, 154.63, (C-aryl), 192.97 (C=O).
FT-IR (KBr): ν [cm⁻¹]=1661, 1588, 1454, 1295, 1260, 1195, 1086, 944, 907, 838. 805.

The invention is not limited to the embodiments hereinbefore described which may be varied in detail.

## Claims

1. A process for the preparation of benzocycloheptapyridine-11-ones of the general formula B: 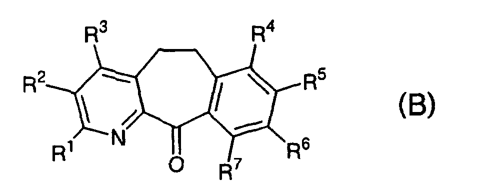 wherein R¹, R², R³, R⁴, R⁵, R⁶ and R⁷ are independently selected from one or more of the following groups:
hydrogen, halogen, hydroxy, C₁-C₆-alkyl, C₁-C₆-alkoxy, nitro, CF₃, thio-C₁-C₆-alkyl.
and pharmaceutically acceptable salts thereof,
comprising the steps of:-
deprotoriation of a compound of the formulae V or VI: 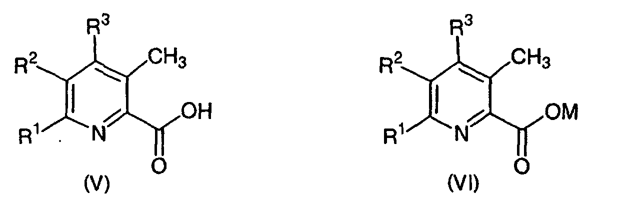 wherein R¹, R² and R³ are as defined above, and M is an alkali metal ion,
to form a dianion;
alkylation of the dianion to form a coupling intermediate of the formula: 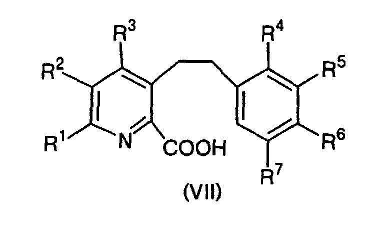 wherein R¹, R², R3, R⁴, R5, R⁶ and R⁷ are as defined above; and
converting the coupling intermediate of the formula VII into a compound of formula B by forming an acid chloride and carrying out a Friedel Crafts reaction.

2. A process as claimed in claim 1 wherein deprotonation is carried out using a lithium deprotonation agent, preferably lithium amide or n-butyl lithium.

3. A process as claimed in claim 1 or 2 wherein deprotonation is carried out by reaction of a compound of formula VI as defined in claim 1 with one equivalent of the deprotonation agent.

4. A process as claimed in claim 1 or 2 wherein deprotonation is carried out by reaction of a compound of formula V as defined in claim 1 with two equivalents of the deprotonation agent.

5. A process as claimed in any of claims 2 to 4 wherein the lithium amide is lithium diisopropylamide.

6. A process as claimed in any preceding claim wherein the dianions are picolinic acid dianions generated from picolinic acid or salts thereof, especially the lithium salt.

7. A process as claimed in any preceding claim wherein the reaction is carried out within a temperature range of from -50°C to 70°C, preferably from -15°C to 20°C.

8. A process as claimed in any preceding claim wherein the process is carried out in the presence of a solvent, preferably a donor solvent, especially tetrahydrofuran.

9. A process as claimed in any preceding claim wherein alkylation involves the addition of benzylhalide to the dianion.

10. A process as claimed in any preceding claim wherein the alkylation reaction is carried out within a temperature range of from -50°C to 70°C, preferably from -15°C to 20°C.

11. A process as claimed in any preceding claim wherein the alkylation process is carried out in the presence of a solvent, preferably a donor solvent, especially tetrahydrofuran.

12. A process for preparing (benzocycloheptapyridinylidene) piperidine derivatives of formula A: 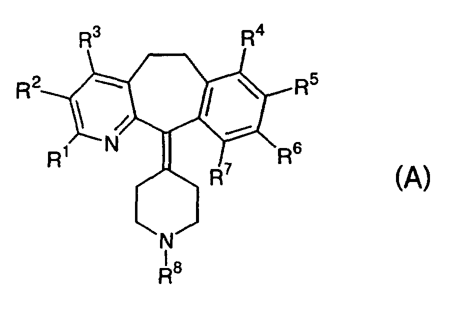 wherein R¹, R², R3, R⁴, R⁵, R⁶ and R⁷ are as defined in claim 1 and R⁸ is selected from one or more of:
hydrogen, C₁-C₆-alkyl or COOR⁹ (wherein R⁹ may be C₁-C₆ -alkyl, C₁-C₆ - alkenyl, phenyl, benzyl), CO-aryl, CO-heteroaryl, CO-alkyl, CO-alkyl-aryl.
including the step of preparing a compound of formula B by a process as claimed in any of claims 1 to 11.

## Patentansprüche

1. Verfahren zur Herstellung von Benzocycloheptapyridin-1 11-onen der allgemeinen Formel B: 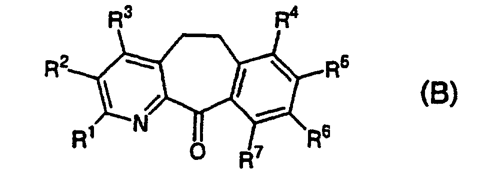 worin R¹, R², R³, R⁴. R⁵, R⁶ und R⁷ unabhängig aus einer oder mehreren der folgenden Gruppe(n) ausgewählt sind:
Wasserstoff, Halogen, Hydroxy, C₁-C₆-Alkyl, C₁-C₆-Alkoxy, Nitro, CF₃, Thio-C₁-C₆-Alkyl;
und pharmazeutisch verträgliche Salze davon, umfassend die Schritte von:
Deprotonierung einer Verbindung der Formeln V oder VI: 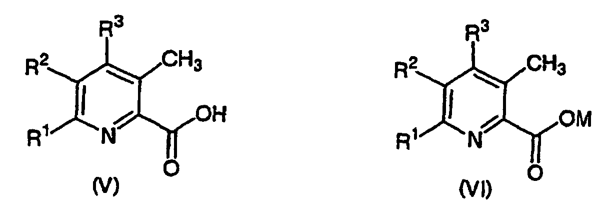 worin R¹, R² und R³ wie oben definiert sind, und M ein Alkalimetallion darstellt,
zur Bildung eines Dianions;
Alkylierung des Dianions zur Bildung eines Kopplungs-Intermediärprodukts der Formel: 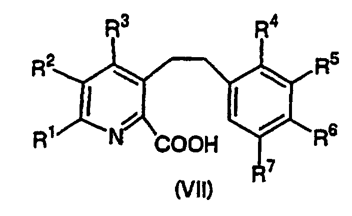 worin R¹ R², R³, R⁴, R⁵, R⁶ und R⁷ wie oben definiert sind; und
Umwandlung des Kopplungs-Intermediärprodukts der Formel VII in eine Verbindung der Formel B durch Bildung eines Säurechlorids und Durchführung einer Friedel-Crafts-Reaktion.

2. Verfahren nach Anspruch 1, worin die Deprotonierung unter Verwendung eines Lithium-Deprotonierungsmittels, bevorzugt eines Lithiumamids oder n-Butyllithiums, durchgeführt wird.

3. Verfahren nach Anspruch 1 oder 2, worin die Deprotonierung durch Reaktion einer Verbindung der Formel VI wie nach Anspruch 1 definiert, mit einem Äquivalent des Deprotonierungsmittels durchgeführt wird.

4. Verfahren nach Anspruch 1 oder 2, worin die Deprotonierung durch Reaktion einer Verbindung der Formel V wie nach Anspruch 1 definiert, mit zwei Äquivalenten des Deprotonierungsmittels durchgeführt wird.

5. Verfahren nach einem der Ansprüche 2 bis 4, worin das Lithiumamid Lithiumdiisopropylamid ist.

6. Verfahren nach einem der vorangehenden Ansprüche, worin die Dianionen Picolinsäure-Dianionen sind, die aus Picolinsäure oder Salzen davon, besonders dem Lithiumsalz, gebildet sind.

7. Verfahren nach einem der vorangehenden Ansprüche, worin die Reaktion in einem Temperaturbereich von -50 °C bis 70 °C, bevorzugt von -15 °C bis 20 °C durchgeführt wird.

8. Verfahren nach einem der vorangehenden Ansprüche, worin das Verfahren in Anwesenheit eines Lösungsmittels, bevorzugt eines Donatorlösungsmittels, besonders Tetrahydrofuran, durchgeführt wird.

9. Verfahren nach einem der vorangehenden Ansprüche, worin die Alkylierung das Zufügen von Benzylhalogenid zum Dianion beinhaltet.

10. Verfahren nach einem der vorangehenden Ansprüche, worin die Alkylierungsreaktion in einem Temperaturbereich von -50 °C bis 70 °C, bevorzugt von -15 °C bis 20 °C, durchgeführt wird.

11. Verfahren nach einem der vorangehenden Ansprüche, worin das Alkylierungsverfahren in Anwesenheit eines Lösungsmittels, bevorzugt eines Donatorlösungsmittels, besonders Tetrahydrofuran, durchgeführt wird.

12. Verfahren zur Herstellung von (Benzocycloheptapyridinyliden)piperidin-Derivaten der Formel A: 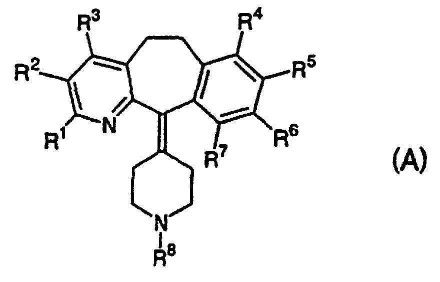 worin R¹, R², R³, R⁴, R⁵, R⁶ und R⁷ wie nach Anspruch 1 definiert sind und R⁸ aus einem oder mehreren des Folgenden ausgewählt ist:
Wasserstoff, C₁-C₆-Alkyl oder COOR⁹ (worin R⁹ C₁-C₆-Alkyl C₁-C₆-Alkenyl, Phenyl, Benzyl darstellen kann), CO-Aryl, CO-Heteroaryl, CO-Alkyl, CO-Alkyl-Aryl;
einschließlich des Schrittes zur Herstellung einer Verbindung der Formel B durch ein Verfahren nach einem der Ansprüche 1 bis 11.

## Revendications

1. Procédé de préparation de benzocycloheptapyridine-11-ones de formule générale B : 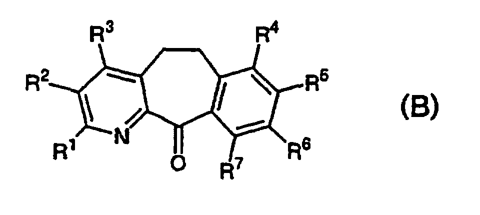 dans laquelle R¹, R², R³, R⁴, R⁵, R⁶ et R⁷ sont sélectionnés de manière indépendante parmi un ou plusieurs des groupes suivants :
hydrogène, halogène, hydroxy, alkyle en C₁-C₆, alkoxy en C₁-C₆, nitro, CF₃, thio-alkyle en C₁-C₆,
et les sels pharmaceutiquement acceptables de ceux-ci,
comprenant les étapes de :
déprotonation d'un composé ayant les formules V ou VI : 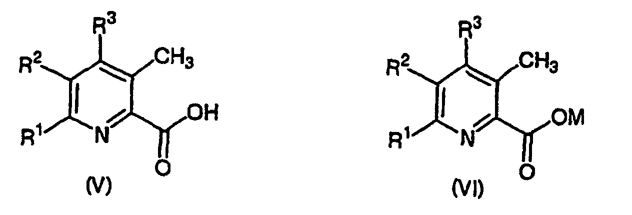
dans laquelle R¹, R² et R³ sont tels que définis ci-dessus et M est un ion de métal alcalin,
pour former un dianion ;
alkylation du dianion pour former une forme active intermédiaire de couplage de formule : 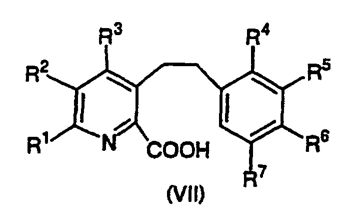 dans laquelle R¹, R², R³, R⁴, R⁵, R⁶ et R⁷ sont tels que définis ci-dessus ; et
conversion de la forme active intermédiaire de couplage ayant la formule VII en un composé de formule B en formant un chlorure d'acide et en effectuant une réaction de Friedel Crafts.

2. Procédé selon la revendication 1, dans lequel la déprotonation est effectuée en utilisant un agent de déprotonation au lithium, de préférence un amide de lithium ou du n-butyl lithium.

3. Procédé selon la revendication 1 ou 2, dans lequel la déprotonation est effectuée par la réaction d'un composé de formule VI tel que défini dans la revendication 1 avec un équivalent de l'agent de déprotonation.

4. Procédé selon la revendication 1 ou 2, dans lequel la déprotonation est effectuée par réaction d'un composé de formule V tel que défini dans la revendication 1 avec deux équivalents de l'agent de déprotonation.

5. Procédé selon l'une quelconque des revendications 2 à 4, dans lequel l'amide de lithium est le diisopropylamide de lithium.

6. Procédé selon l'une quelconque des revendications précédentes, dans lequel les dianions sont des dianions d'acide picolinique générés à partir d'acide picolinique ou des sels de celui-ci, en particulier le sel de lithium.

7. Procédé selon l'une quelconque des revendications précédentes, dans lequel la réaction est effectuée dans une plage de température allant de - 50 °C à 70 °C, de préférence de -15 °C à 20 °C.

8. Procédé selon l'une quelconque des revendications précédentes, dans lequel le procédé est effectué en présence d'un solvant, de préférence un solvant donneur, en particulier le tétrahydrofurane.

9. Procédé selon l'une quelconque des revendications précédentes dans lequel l'alkylation comporte l'ajout d'halogénure de benzyle au dianion.

10. Procédé selon l'une quelconque des revendications précédentes dans lequel la réaction d'alkylation est effectuée dans une plage de température allant de - 50 °C à 70 °C, de préférence de - 15 °C à 20 °C.

11. Procédé selon l'une quelconque des revendications précédentes dans lequel le procédé d'alkylation est effectué en présence d'un solvant, de préférence un solvant donneur, en particulier le tétrahydrofurane.

12. Procédé de préparation de dérivés de pipéridine de benzocycloheptapyridinylidène de formule A : 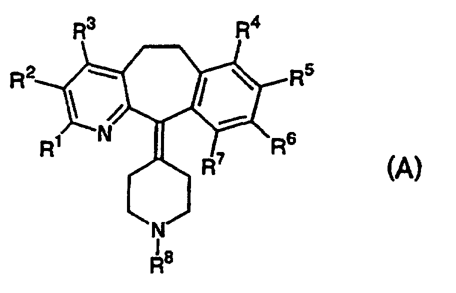 dans laquelle R¹, R², R³, R⁴, R⁵, R⁶ et R⁷ sont tels que définis dans la revendication 1 et R⁸ est sélectionné parmi un ou plusieurs de :
hydrogène, alkyle en C₁-C₆ ou COOR⁹ (dans lequel R⁹ peut être alkyle en C₁-C₆, alkényle en C₁-C₆, phényle, benzyle), CO-aryle, CO-hétéroaryle, CO-alkyle, CO-alkylearyle,
comprenant l'étape consistant à préparer un composé de formule B par un procédé selon l'une quelconque des revendications 1 à 11.
